# EUROPÄISCHE PATENTSCHRIFT

(11) **EP 2 053 996 B1**
(45) Veröffentlichungstag und Bekanntmachung des Hinweises auf die Patenterteilung: **02.10.2013**
(21) Anmeldenummer: 07763734.6
(22) Anmeldetag: 04.07.2007
(51) Int. Cl.: A61F 5/00

(54) **EINRICHTUNG ZUR BEHANDLUNG VON FETTLEIBIGKEIT**
DEVICE FOR TREATING OBESITY
DISPOSITIF DE TRAITEMENT DE L'OBÉSITÉ

(30) Priorität: 21.08.2006 AT 13942006
(43) Veröffentlichungstag der Anmeldung: 06.05.2009
(73) Patentinhaber: A.M.I. Agency for Medical Innovations GmbH, 6800 Feldkirch (AT)
(72) Erfinder: KIERATH, Tony, West Perth, W.A. 6005 (AU); EGLE, Walter, 6842 Koblach (AT)
(74) Vertreter: Hofmann, Ralf U.
(86) Internationale Anmeldenummer: PCT/AT2007/000334
(87) Internationale Veröffentlichungsnummer: WO 2008/022360

(56) Entgegenhaltungen:
- EP-A- 1 669 045
- WO-A-02/053040
- US-A1- 2004 260 316
- US-A1- 2004 267 377

## Beschreibung

Die Erfindung betrifft eine Einrichtung zur Behandlung von Fettleibigkeit, welche ein ringförmig um den Magen legbares Band mit einer Verschlusseinrichtung zum Schließen des Bandes zu einem Ring und ein mit dem Band verbundenes oder verbindbares Hüllteil zum Umhüllen eines an das angelegte Band anschließenden Magenbereichs umfasst.

Einrichtungen zur Behandlung von Fettleibigkeit mittels eines ringförmig um den Magen legbaren Bandes, das durch eine Verschlusseinrichtung zu einem Ring verschließbar ist, sind in unterschiedlichen Ausführungsformen bekannt und werden meist als Magenbänder bezeichnet. Beispielsweise geht ein derartiges Magenband aus der EP 0 702 529 B1 hervor. Das ringförmig um den Magen legbare Band besitzt eine längsverlaufende innere Hohlkammer und an den beiden Enden des Bandes sind erste und zweite Verschlussteile angebracht, die zum Schließen des Bandes miteinander verrastbar sind. Zur Verengung des Durchlassquerschnittes der Magenbandöffnung des geschlossenen Magenbandes wird die Hohlkammer mit der gewünschten Menge eines Füllmediums befüllt. Diese Befüllung erfolgt über einen mit dem Magenband über ein Röhrchen verbundenen Injektionsport, der unter die Haut des Patienten implantiert wird. Weitere Magenbänder mit einstellbaren Durchlassquerschnitten gehen beispielsweise aus der US 2004/0158272 A1, der US 2004/0260319 A1 und der US 2004/0267377 A1 hervor.

Magenbänder werden üblicherweise in der Nähe des Eingangs der Speiseröhre um den Magen gelegt. Wird ein Magenband weiter unten gesetzt, so ist das verbleibende Magenreservoir oberhalb des Bandes relativ groß und der Magen kann in diesem Bereich zudem dilatieren, so dass der gewünschte Gewichtsverlust nicht erreicht wird. Wenn der Magen wie üblich hoch oben in der Nähe des Eingangs der Speiseröhre verengt wird, so können die vom Patienten aufgenommenen Speisen nur langsam durch diesen verengten Bereich treten. Es kann daher zu einer Ansammlung von aufgenommener Nahrung im Bereich der distalen Speiseröhre (=distaler Esophagus) kommen. Die distale Speiseröhre wird somit entgegen ihrer natürlichen Funktion als Reservoir für Speisen verwendet. Als mögliche Langzeitkomplikation kann es zu einer Dilatation der distalen Speiseröhre kommen, worauf das Magenband entfernt werden muss.

Ein Magenband der eingangs genannten Art ist aus der EP 1 669 045 A1 bekannt. Um eine Erweiterung eines proximal zum Magenband liegenden Bereichs des Magens zu verhindern, ist am Band ein nach proximal seitlich abstehendes Hüllteil befestigt. Im implantierten Zustand des Bandes umgibt das Hüllteil einen proximal des Magenbandes liegenden Bereich des Magens manschettenartig und stützt die von ihm umhüllte Magenwand elastisch ab.

Aufgabe der Erfindung ist es, eine verbesserte Einrichtung der eingangs genannten Art bereitzustellen, bei der der Magen als Reservoirorgan genutzt werden kann und die eine hohe Effektivität aufweist. Erfindungsgemäß gelingt dies durch eine Einrichtung mit den Merkmalen des Anspruchs 1.

Durch die Erfindung wird somit ein mindestens zweistufiges Magenband bereitgestellt. Das erste und das zweite zu einem Ring geschlossene Band stellen jeweils eine Bremse für die Speisen dar und das zwischen dem ersten und dem zweiten Band liegende, den Magen manschettenartig umgebende Hüllteil wirkt einer Dilatation des Magens im Bereich zwischen dem ersten und dem zweiten Band entgegen bzw. begrenzt diese. Es kann dadurch eine effektive Abbremsung der vom Patienten zu sich genommenen Speisen mit einem raschen Sättigungsgefühl erreicht werden, wobei dennoch der Magen im Bereich zwischen dem zweiten und dem ersten Band als Reservoir genutzt werden kann.

Das erste Band kann beispielsweise um den proximalen Magen in der Nähe des Eingangs der Speiseröhre angelegt werden, um für die geschluckten Speisen eine erste Bremse zu bilden. Das distal dieses ersten Bandes angelegte zweite Band kann günstigerweise einen gegenüber dem ersten Band kleineren inneren Durchmesser aufweisen bzw. auf einen solchen kleinen inneren Durchmesser eingestellt sein, um gegenüber dem ersten Band eine stärkere Verengung auszubilden.

In einer vorteilhaften Ausführungsform der Erfindung sind nur dieses erste und zweite Band vorhanden. In einer weiteren Ausführungsform der Erfindung kann ein weiteres distal des zweiten Bandes anlegbares drittes Band vorgesehen sein, wobei auch der Bereich des Magens zwischen dem dritten und dem zweiten Band vom Hüllteil bzw. einem weiteren zwischen dem zweiten und dem dritten Band sich erstreckenden Hüllteil umhüllt wird. Es kann dadurch eine weitere Bremsstufe bzw. ein weiteres nutzbares Magenreservoir zwischen dem dritten Band und dem zweiten Band bereitgestellt werden. Weitere Bänder mit jeweils dazwischenliegenden umhüllten Abschnitten des Magens sind ebenfalls denkbar und möglich.

In einer bevorzugten Ausführungsform der Erfindung sind das erste und das zweite Band und das mit dem ersten und dem zweiten Band verbundene Hüllteil als herstellerseitig vorgefertigte Einheit ausgebildet, deren Teile miteinander verbunden sind. Diese vorgefertigte Einheit ist als Ganzes in den Körper des Patienten implantierbar. Das Hüllteil ist hierbei vorzugsweise untrennbar mit dem ersten und zweiten Band verbunden, d.h. nicht ohne Zerstörung der vorgefertigten Einheit entfernbar. Durch die Ausbildung als vorgefertigte Einheit wird eine einfache und zuverlässige Implantation erreicht, wobei die Gefahr von operativen Fehlern verringert wird. Beispielsweise kann das Hüllteil an den ersten und zweiten Bändern angeklebt sein. Auch eine einstückige Ausbildung des Hüllteils mit dem ersten und/oder dem zweiten Band oder Teilen hiervon ist denkbar und möglich.

Andererseits ist es auch denkbar und möglich, das Hüllteil mit dem ersten und/oder zweiten Band über Verschlusselemente zu verbinden, die einerseits am Hüllteil andererseits am ersten und/oder zweiten Band angeordnet sind, beispielsweise in Form von miteinander verrastbaren Verschlusselementen. Eine Verbindung des Hüllteils mit dem ersten und/oder zweiten Band könnte in diesem Fall auch erst bei der Implantation der Einrichtung erfolgen. Denkbar und möglich ist es weiters, das Hüllteil mit dem ersten und/oder zweiten Band im Zuge der Implantation zu vernähen.

Es ist bevorzugt, dass der innere Durchmesser des ersten und zweiten Bandes einstellbar ist, wenn diese zu einem Ring geschlossen sind. Vorzugsweise weisen hierzu das erste und zweite Band jeweils mindestens eine mit einem Füllmedium befüllbare innere Hohlkammer auf, die sich in Längsrichtung des Bandes zumindest über den Grossteil von dessen Länge erstreckt. Der innere Durchmesser des Bandes kann hierbei durch die Menge des eingebrachten Füllmediums reguliert werden, wie dies bei Magenbändern hinlänglich bekannt ist. Denkbar und möglich wäre es beispielsweise auch, den inneren Durchmesser des ersten und/oder zweiten Bandes durch eine zwischen den Längsenden des Bandes wirkende Kolben-Zylinder-Einheit zu regulieren, wie dies ebenfalls bereits bekannt ist.

Weitere Vorteile und Einzelheiten der Erfindung werden im Folgenden anhand der beiliegenden Zeichnung erläutert. In dieser zeigen:
- Fig. 1: ein erstes Ausführungsbeispiel der Erfindung im geöffneten Zustand des ersten und zweiten Bandes, in Schrägsicht;
- Fig. 2: eine schematische Darstellung der um den Magen angelegten Einrichtung;
- Fig. 3: einen Längsschnitt durch eines der beiden Bänder (wobei die Zuglasche des ersten Verschlussteils abgeschnitten ist);
- Fig. 4: eine Schrägsicht der Endabschnitte eines der beiden Bänder mit den Verschlussteilen im geöffneten Zustand des Bandes (der Übersichtlichkeit halber ohne die Zuglasche des ersten Verschlussteils);
- Fig. 5 und Fig. 6: Schrägsichten von etwas modifizierten Ausführungsformen der Erfindung;
- Fig. 7: eine schematische Schnittdarstellung einer um den Magen angelegten Einrichtung gemäß einer weiteren Ausführungsvariante der Erfindung;
- Fig. 8: eine Ansicht eines Abschnitts des Hüllteils gemäß einer weiteren Ausführungsvariante der Erfindung;
- Fig. 9 und Fig. 10: ein Ausführungsbeispiel eines dreistufigen Magenbandes gemäß der Erfindung im geöffneten und geschlossenen Zustand der Bänder, in Schrägsicht;
- Fig. 11: eine weitere Ausführungsform der Erfindung im geöffneten Zustand der Bänder, in Schrägsicht;
- Fig. 12: eine schematische Darstellung eines Abschnitts eines Bandes und eines Hüllteils gemäß einer weiteren Ausführungsvariante der Erfindung.

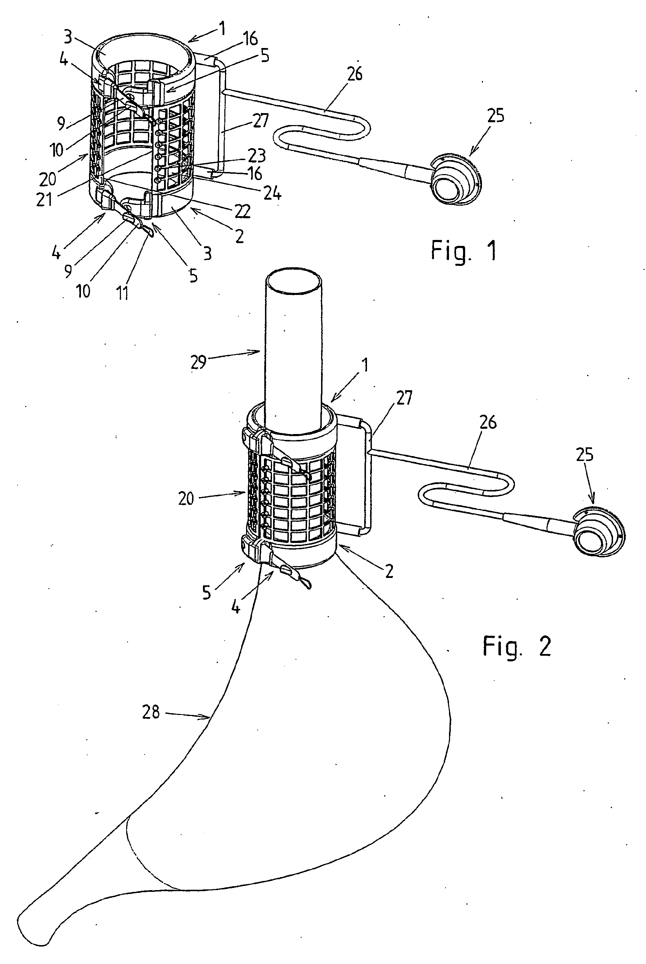

Ein erstes Ausführungsbeispiel der Erfindung ist in den Fig. 1 bis 4 dargestellt. Die erfindungsgemäße Einrichtung umfasst bei diesem Ausführungsbeispiel ein erstes und ein zweites Band 1, 2, die in gleicher Weise ausgebildet sind, wobei Teile des ersten und zweiten Bandes 1, 2 mit den gleichen Bezugszeichen bezeichnet sind. Ein jeweiliges Band 1, 2 umfasst einen Körperabschnitt 3, an dessen beiden Enden ein erstes und ein zweites Verschlussteil 4, 5 angebracht sind. Das erste Verschlussteil 4 weist einen Fortsatz 6 mit einem Rastvorsprung 7 auf. Am vom Körperabschnitt 3 abgelegenen Ende des ersten Verschlussteils 4 ist eine Zuglasche 9 mit einem Vorrastvorsprung 10 und einer Einhängeschlaufe 11 angeordnet.

Das zweite Verschlussteil 5 besitzt einen ösenförmigen Abschnitt 12, an weichem eine Zuglasche 13 angeordnet ist.

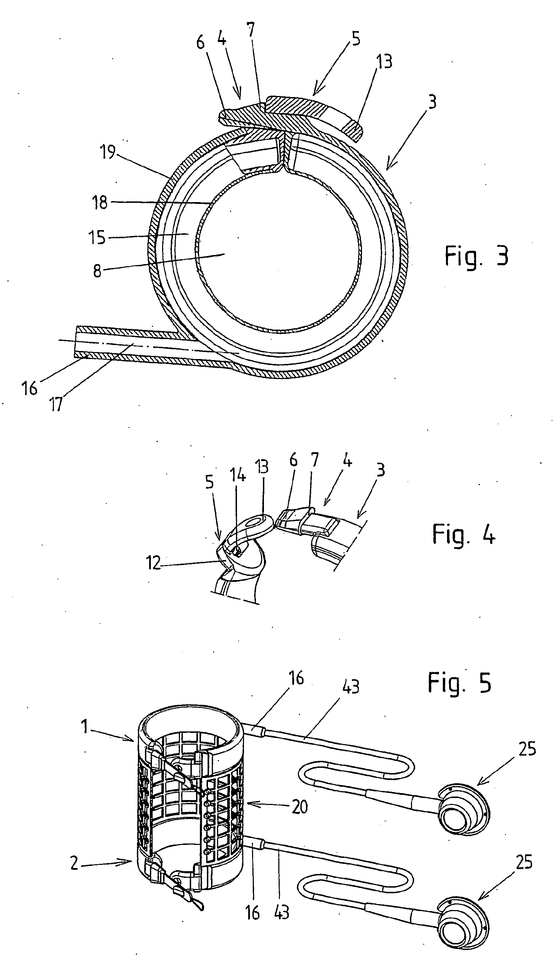

Zum Schließen des jeweiligen Bandes 1, 2 zu einem Ring mit einer Durchlassöffnung 8 wird das erste Verschlussteil 4 durch die Öffnung 14 des ösenförmigen Abschnitts 12 des zweiten Verschlussteils 5 gezogen, bis der Rastvorsprung 7 einrastet. Der Vorrastvorsprung 10 erleichtert das Schließen des Magenbandes, indem nach dem Durchziehen der Zuglasche 9 durch die Öffnung 14 bis zum Einrasten des Vorrastvorsprungs 10 ein teilweise geschlossener Zustand bereitgestellt wird. Nach dem Schließen des Bandes 1, 2 kann die Zuglasche 9 abgeschnitten werden, wie in Fig. 3 dargestellt.

Die Verschlusseinrichtung zum Schließen des jeweiligen Bandes 1, 2 kann in unterschiedlicher Weise modifiziert werden.

Der Körperabschnitt 3 eines jeweiligen Bandes 1, 2 besitzt eine über seine Länge durchgehende innere Hohlkammer 15. Der Körperabschnitt 3 wird hierbei von einem schlauchartigen Element gebildet, welches an seinen beiden Enden durch eingeklebte Verschlüsse verschlossen ist, an denen die Verschlussteile 4, 5 angebracht sind. Der Körperabschnitt 3 ist weiters mit einem Anschlussröhrchen 16 versehen, dessen innerer Kanal 17 mit der Hohlkammer 15 des Körperabschnitts 3 kommuniziert.

Im gezeigten Ausführungsbeispiel ist das Anschlussröhrchen 16 in einem von den Verschlussteilen 4, 5 abgelegenen Bereich am Körperabschnitt 3 angeordnet. Denkbar und möglich wäre es auch, dass das Anschlussröhrchen 16 beispielsweise durch das erste Verschlussteil 4 verläuft, wie dies bekannt ist.

Vorzugsweise liegen die Durchlassöffnungen 8 der zu Ringen geschlossenen Bänder 1, 2 koaxial zueinander (im entspannten Zustand der Einrichtung).

Wenn die innere Hohlkammer 15 durch das Anschlussröhrchen 16 mit einem Füllmedium, beispielsweise steriles Wasser oder Röntgenkontrastmittel, befüllt wird, so kann der innere Durchmesser des zu einem Ring geschlossenen Bandes 1, 2 variiert werden, wie dies ebenfalls bekannt ist. Hierbei dehnt sich die Innenwand 18 des Körperabschnitts 3 unter Verringerung des Durchmessers der Durchlassöffnung 8 des zu einem Ring geschlossenen Bandes 1, 2 in Richtung zur zentralen Längsachse des Rings aus. Die Innenwand 18 weist hierbei eine größere Elastizität als die Außenwand 19 des Körperabschnitts 3 auf, welche beispielsweise durch eine Gewebeeinlage verstärkt sein kann.

Vorzugsweise bestehen die Bänder 1, 2 zumindest im Wesentlichen, also beispielsweise abgesehen von Gewebeeinlagen, aus Silikon.

Die Einrichtung umfasst weiters ein Hüllteil 20. Dieses erstreckt sich zwischen den im Abstand zueinander angeordneten Bändern 1, 2. Das Hüllteil 20 ist hierbei im Wesentlichen über die Länge des jeweiligen Bandes 1, 2 (über die Länge des Bandes abgesehen von den Verschlussteilen 4, 5) bzw. über die Länge des Körperabschnitts 3 des jeweiligen Bandes 1, 2 mit dem jeweiligen Band 1, 2 verbunden. Im gezeigten Ausführungsbeispiel ist es hierbei an den Bändern 1, 2 an deren einander zugewandten Seitenrändern angeklebt.

Wenn die Bänder 1, 2 zu Ringen geschlossen sind, liegen die Umfangsenden 21, 22 des Hüllteils 20 benachbart zueinander. Diese Umfangsenden 21, 22 sind mit Nähösen 23 versehen, durch welche sie miteinander vernäht werden können.

Stattdessen könnten die an die Umfangsenden 21, 22 anschließenden Abschnitte des Hüllteils 20 im geschlossenen Zustand der Bänder 1, 2 auch einander überlappen.

Das Hüllteil 20 besteht vorzugsweise aus Silikon. Es kann hierbei elastisch ausgebildet sein. Denkbar und möglich ist es auch, in das Silikon Verstärkungsmaterial, insbesondere Verstärkungsfasern einzubetten.

Im Ausführungsbeispiel gemäß den Fig. 1 bis 4 ist das Hüllteil 20 gitterartig bzw. netzartig ausgebildet, wobei es eine Vielzahl von über seine Ausdehnung verteilten Durchtrittsöffnungen 24 aufweist.

Im Ausführungsbeispiel gemäß den Fig. 1 bis 4 sind die Bänder 1, 2 über einen gemeinsamen Injektionsport 25 befüllbar, wobei der Injektionsport 25 über Verbindungsröhrchen 26, 27 mit den Anschlussröhrchen 16 der Bänder 1, 2 verbunden ist.

Der implantierte Zustand der Einrichtung ist in Fig. 2 schematisch dargestellt. Das erste Band 1 wird um den Magen 28 in der Nähe des Eingangs der Speiseröhre 29 oder um die Speiseröhre 29 in der Nähe ihres Eintritts in den Magen 28 oder im Übergangsbereich zwischen Speiseröhre 29 und Magen 28 angelegt. Das zweite Band 2 wird weiter distal, d.h. vom Mund weiter entfernt, um den Magen 28 angelegt. Der zwischen dem ersten und zweiten Band 1, 2 liegende Bereich des Magens 28 wird vom Hüllteil 20 umhüllt. Vorzugsweise werden die Umfangsenden 21, 22 des Hüllteils 20 durch die Nähösen 23 miteinander vernäht, was in Fig. 2 der Übersichtlichkeit halber nicht dargestellt ist. Weiters werden die Bänder 1, 2 mit der gewünschten Menge von Füllmedium befüllt, um die Durchlassöffnungen 8 der Bänder 1, 2 auf die gewünschte Größe einzustellen. Die Befüllung erfolgt in diesem Ausführungsbeispiel durch einen gemeinsamen Injektionsport 25 und die Durchlassöffnungen 8 der Bänder 1, 2 werden gemeinsam und miteinander gekoppelt eingestellt.

Vorzugsweise ist die Durchlassöffnung 8 des zweiten Bandes 2 kleiner als die des ersten Bandes 1. Dies kann beispielsweise dadurch erreicht werden, dass der Körperabschnitt 3 des zweiten Bandes 2 kürzer als derjenige des ersten Bandes 1 ist. Stattdessen könnte die Innenwand 18 des zweiten Bandes 2 auch leichter dehnbar als die Innenwand 18 des ersten Bandes 1 ausgebildet sein, beispielsweise mittels unterschiedlicher Materialstärken und/oder durch unterschiedliche Materialien. Auch eine Kombination dieser Maßnahmen ist möglich. Geschluckte Speisen können dadurch das erste Band 1 leichter passieren als das zweite Band 2.

Da die inneren Hohlkammern 15 der Bänder 1, 2 über das Verbindungsröhrchen 27 miteinander kommunizieren, kommt es zudem, wenn ein Bolus (=Inhalt eines Schlucks) durch die Durchlassöffnung 8 des ersten Bandes 1 gedrückt wird, zu einem Druckanstieg im zweiten Band 2, so dass sich dessen Durchlassöffnung 8 verengt. Die Speise wird dadurch vom zweiten Band stärker gebremst. Wenn andererseits Speise durch das zweite Band 2 gedrückt wird, so steigt der Druck im ersten Band 1, wodurch die Nachführung von Speise gebremst wird.

Insgesamt kommt es zu einer zweistufigen Bremsung von zugeführter Speise, bis diese den unterhalb des zweiten Bandes 2 liegenden Hauptbereich des Magens erreicht. Die Geschwindigkeit, mit der Speisen zugeführt werden können, wird dadurch verringert. Durch im Bereich zwischen dem zweiten Band 2 und dem ersten Band 1 angestaute Nahrung kommt es weiters zu einem Sättigungsgefühl des Patienten.

Der zwischen dem zweiten Band 2 und ersten Band 1 liegende Bereich des Magens 28 kann als Reservoir für Speise, welche das erste Band 1, nicht aber das zweite Band 2 passiert hat, genutzt werden. Durch das Hüllteil 20 wird eine Dilatation dieses Bereichs des Magens dabei verhindert bzw. begrenzt.

Das in Fig. 5 dargestellte Ausführungsbeispiel entspricht dem zuvor beschriebenen Ausführungsbeispiel mit dem Unterschied, dass für jedes Band 1, 2 ein eigener Injektionsport 25 vorhanden ist, der mit der Hohlkammer 15 eines jeweiligen Bandes über ein Verbindungsröhrchen 43 verbunden ist, wobei auch die Hohlkammern 15 der beiden Bänder 1, 2 nicht miteinander kommunizieren. Die Durchlassöffnungen 8 der Bänder 1, 2 können somit unabhängig voneinander eingestellt werden. Die beiden Injektionsports 25 können wenn gewünscht an unterschiedlichen Körperstellen implantiert werden, um für den behandelnden Arzt eine einfache Unterscheidung zu ermöglichen.

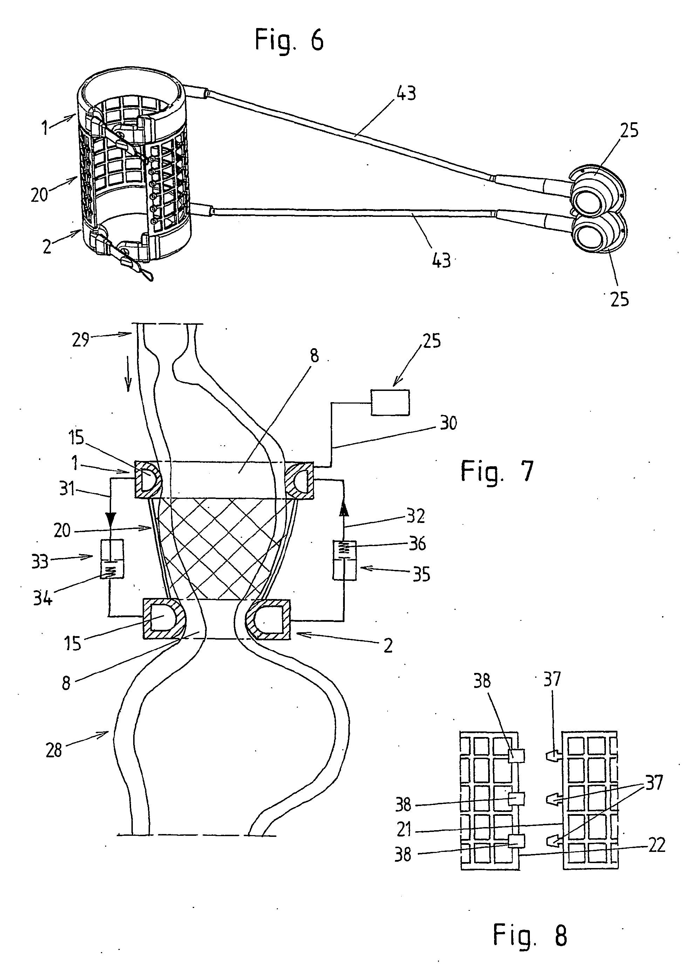

Das Ausführungsbeispiel gemäß Fig. 6 unterscheidet sich von dem in Fig. 5 dargestellten Ausführungsbeispiel nur dadurch, dass die beiden Injektionsports 25 zu einer Einheit miteinander verbunden sind.

In Fig. 7 ist ein weiteres Ausführungsbeispiel schematisch im implantierten Zustand dargestellt. Dieses Ausführungsbeispiel entspricht dem anhand der Fig. 1 bis 4 erläuterten Ausbildungsform mit folgenden Unterschieden: Der Injektionsport 25 kommuniziert über ein Verbindungsröhrchen 30 mit der inneren Hohlkammer 15 des ersten Bandes 1. Die inneren Hohlkammern 15 des ersten und des zweiten Bandes 1, 2 sind durch erste und zweite Verbindungsleitungen 31, 32 miteinander verbunden. In der ersten Verbindungsleitung 31 ist ein Rückschlagventil 33 angeordnet, welches bei einer Strömung in Richtung vom ersten Band 1 zum zweiten Band 2 öffnet, wenn eine Schließkraft F1 überschritten wird. Ein diese Schließkraft bewirkendes Federelement 34 ist schematisch eingezeichnet. In der Verbindungsleitung 32 ist ein bei einer Strömung in Richtung vom zweiten Band 2 zum ersten Band 1 öffnendes Rückschlagventil 35 angeordnet, welches öffnet, wenn eine Schließkraft F2 überwunden wird. Ein diese Schließkraft F2 bewirkendes Federelement 36 ist wiederum schematisch angedeutet.

Die Schließkraft F1 ist kleiner als die Schließkraft F2. Die Bänder 1, 2 können in gleicher Weise ausgebildet sein, also die gleiche Länge und die gleiche Elastizität ihrer Innenwände 18 aufweisen. Andererseits können die Längen der Bänder 1, 2 und/oder die Elastizitäten der Innenwände 18 auch unterschiedlich sein.

Wenn Speise durch die Durchlassöffnung 8 des ersten Bandes 1 durchgedrückt wird und es hierbei zu einem so großen Druckanstieg des Füllmediums kommt, dass die Schließkraft F1 überschritten wird, so kann Füllmedium vom ersten Band 1 in das zweite Band 2 strömen, wobei dessen Durchlassöffnung 8 verkleinert wird. Wenn die Speisemenge zwischen dem ersten Band 1 und dem zweiten Band 2 zunimmt, so steigt der Druck in der Hohlkammer 15 des zweiten Bandes 2, bis schließlich die Schließkraft F2 des Rückschlagventils 35 überschritten wird. In der Folge kann Füllmedium vom zweiten Band 2 zum ersten Band 1 strömen, wobei die Durchlassöffnung 8 des zweiten Bandes 2 vergrößert und die des ersten Bandes 1 verkleinert wird. Für den Patienten wird die Zufuhr von weiterer Speise durch das erste Band 1 damit erschwert, bis sich die Speisemenge im Reservoir zwischen dem ersten und dem zweiten Band 1, 2 wieder verringert hat.

Zum Verschließen des Hüllteils 20 zu einer umfangsgeschlossenen Manschette kann in einer weiteren Ausführungsform der Erfindung eine Verschlusseinrichtung vorhanden sein, beispielsweise in Form eines in Fig. 8 schematisch dargestellten Rastverschlusses. Am einen Umfangsende 21 sind erste Verschlusselemente 37 mit Rastvorsprüngen vorhanden, die in ösenförmige zweite Verschlusselemente 38 am zweiten Umfangsende 22 eingesteckt und mit diesen verrastet werden können.

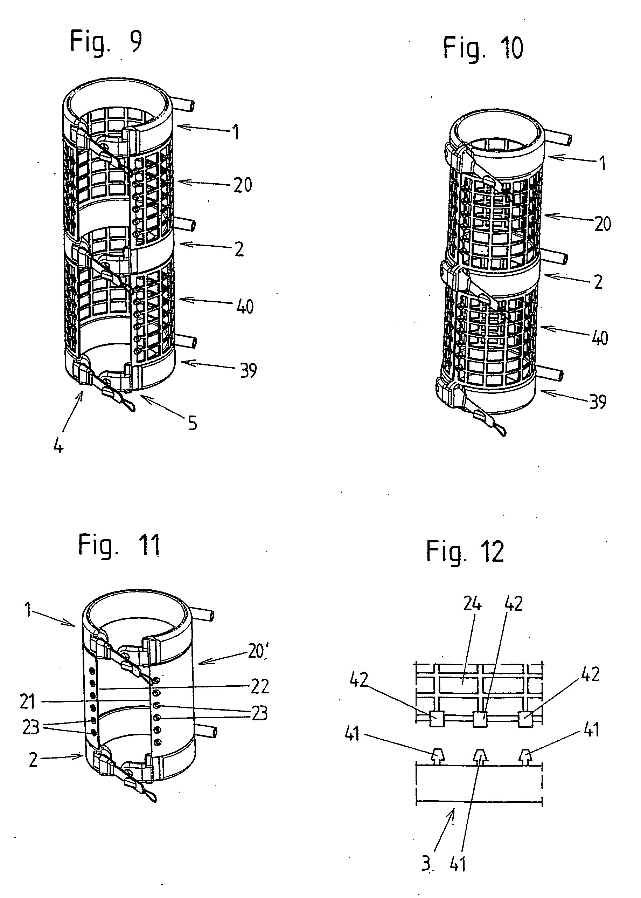

Das in den Fig. 9 und 10 dargestellte weitere Ausführungsbeispiel unterscheidet sich von den zuvor beschriebenen Ausführungsbeispielen dadurch, dass ein drittes Band 39 und ein zweites Hüllteil 40 vorhanden sind, wobei die Einrichtung vorzugsweise wiederum in Form einer vorgefertigten Einheit ausgebildet ist. Das dritte Band 39 ist in analoger Weise wie das erste und das zweite Band 1, 2 ausgebildet, wobei es mittels Verschlussteilen 4, 5 zu einem Ring verschließbar ist, der vorzugsweise koaxial zu den Ringen der geschlossenen ersten und zweiten Bänder 1, 2 liegt (im entspannten Zustand der Einrichtung). Die Durchmesser der einzelnen Bänder 1, 2, 39 im geschlossenen und noch unbefüllten Zustand können gleich sein oder sich unterscheiden. Die Elastizitäten der Innenwände 18 der einzelnen. Bänder 1, 2, 39 können gleich sein oder sich unterscheiden. Wenn die Längen der Bänder 1, 2, 39 und die Elastizitäten der Innenwände 18 gleich sind, so können die Durchlassöffnungen 8 der Bänder 1, 2 39 beispielsweise durch separate Injektionsporte eingestellt werden. Auch Ventileinrichtungen zwischen den einzelnen Bändern 1, 2, 39, analog zum anhand von Fig. 7 beschriebenen Ausführungsbeispiel können vorhanden sein. Im Fall von mehr als zwei Bändern ist es bevorzugt, wenn sich -zumindest solange noch kein durch zugeführte Speise ausgeübter Druck auf die Bänder wirkt - die Durchlassöffnungen der Bänder 1, 2, 39 sich von proximal nach distal zunehmend verkleinern.

Das Hüllteil 40 ist in analoger Weise mit dem zweiten und dritten Band 2, 39 verbunden wie das Hüllteil 20 mit dem ersten und zweiten Band 1, 2 und in analoger Weise wie dieses ausgebildet. Statt dessen könnte auch ein einzelnes durchgehendes Hüllteil vorhanden sein, welches sich über das zweite Band 2 an der Außenseite desselben erstreckt und mit allen drei Bändern 1,2, 39 verbunden ist, vorzugsweise jeweils über im Wesentlichen den gesamten Umfang oder über einzelne Verbindungsstellen, die in Umfangsrichtung und vorzugsweise nicht mehr als 30° voneinander beabstandet sind.

Das in Fig. 11 dargestellte Ausführungsbeispiel unterscheidet sich von dem in den Fig. 1 bis 4 dargestellten Ausführungsbeispiel dadurch, dass anstelle eines gitterartigen Hüllteils 20 ein von einer Folie gebildetes Hüllteil 20' vorhanden ist. Vorzugsweise wird eine Silikonfolie eingesetzt. Im Bereich der beiden Umfangsenden können beispielsweise Nähösen 23 zur Verbindung der beiden Umfangenden 21, 22 vorhanden sein. Auch eine Verschlusseinrichtung, beispielsweise analog zu der in Fig. 8 dargestellten könnte vorhanden sein. Solche folienartigen Hüllteile 20' könnten auch bei den anderen beschriebenen Ausführungsbeispielen eingesetzt werden. Die Folie könnte auch mit einer Vielzahl von Ausstanzungen zur Ausbildung von Durchtrittsöffnungen versehen sein.

Bei den bislang beschriebenen Ausführungsbeispielen war die erfindungsgemäße Einrichtung jeweils als herstellerseitig vorgefertigte Einheit ausgebildet, wobei die Hüllteile 20, 20', 40 untrennbar mit den Bändern 1, 2, 39 verbunden sind. Stattdessen könnten beispielsweise auch Verschlusselemente 41, 42 vorhanden sein, um das Hüllteil 20, 20', 40 jeweils mit zumindest einem der Bänder 1, 2, 39 oder dem jeweiligen Band 1, 2, 39 zu verbinden. Eine solche Ausführungsform ist in Fig. 12 schematisch abschnittsweise dargestellt. Die Rastvorsprünge aufweisenden Verschlusselemente 41 können hierbei in die ösenförmigen Verschlusselemente 42 eingeführt und mit diesen verrastet werden.

Denkbar und möglich wäre es auch, das Hüllteil 20, 20', 40 erst nach dem Einsetzen der Bänder 1, 2, 39 mit zumindest einem der Bänder 1, 2, 39 oder einem jeweiligen Band 1, 2, 39 zu vernähen, was aber weniger bevorzugt ist. Entsprechende Nähösen können hierzu vorgesehen sein.

Unterschiedliche weitere Modifikationen der beschriebenen Ausführungsformen sind denkbar und möglich, ohne den Bereich der Erfindung zu verlassen. Beilspielsweise können in den Hüllteilen 20, 20', 40 Fensteröffnungen vorgesehen sein, um außerhalb der Hüllteile liegendes Gewebe (beispielsweise ein umgeschlagener Abschnitt der Magenwand) mit innerhalb der Hüllteile liegendem Gewebe vernäht werden kann. Die Bänder 1, 2, 39 können jeweils auch mehrere Hohlkammern 15 aufweisen, wie dies bekannt ist. Eine Einstellung des Querschnitts der Durchlassöffnung kann jeweils auch in anderer Form als durch befüllbare Hohlkammern erfolgen, beispielsweise durch Kolben-Zylinder-Einheiten, wie dies ebenfalls bekannt ist.

### Legende zu den Hinweisziffern:

| | | | |
|---|---|---|---|
| 1 | erstes Band | 22 | Umfangsende |
| 2 | zweites Band | 23 | Nähöse |
| 3 | Körperabschnitt | 24 | Durchtrittsöffnung |
| 4 | erstes Verschlussteil | 25 | Injektionsport |
| 5 | zweites Verschlussteil | 26 | Verbindungsröhrchen |
| 6 | Fortsatz | 27 | Verbindungsröhrchen |
| 7 | Rastvorsprung | 28 | Magen |
| 8 | Durchlassöffnung | 29 | Speiseröhre |
| 9 | Zuglasche | 30 | Verbindungsröhrchen |
| 10 | Vorrastvorsprung | 31 | Verbindungsleitung |
| 11 | Einhängeschlaufe | 32 | Verbindungsleitung |
| 12 | ösenförmiger Abschnitt | 33 | Rückschlagventil |
| 13 | Zuglasche | 34 | Federelement |
| 14 | Öffnung | 35 | Rückschlagventil |
| 15 | Hohlkammer | 36 | Federelement |
| 16 | Anschlussröhrchen | 37 | erstes Verschlusselement |
| 17 | Kanal | 38 | zweites Verschlusselement |
| 18 | Innenwand | 39 | drittes Band |
| 19 | Außenwand | 40 | Hüllteil |
| 20, 20' | Hüllteil | 41 | Verschlusselement |
| 21 | Umfangsende | 42 | Verschlusselement |
| | | 43 | Verbindungsröhrchen |

## Patentansprüche

1. Einrichtung zur Behandlung von Fettleibigkeit, welche ein ringförmig um den Magen (28) legbares Band (1, 2) mit einer Verschlusseinrichtung (4, 5) zum Schließen des Bandes (1, 2) zu einem Ring und ein mit dem Band (1, 2) verbundenes oder verbindbares Hüllteil (20, 20') zum Umhüllen eines an das angelegte Band (1, 2) anschließenden Magenbereichs umfasst, **dadurch gekennzeichnet, dass** die Einrichtung zumindest ein erstes und ein zweites Band (1, 2) mit jeweils einer Verschlusseinrichtung (4, 5) zum Schließen des jeweiligen Bandes (1, 2) zu einem Ring umfasst, wobei das mit dem Hüllteil (20, 20') verbundene oder verbindbare erste Band (1) um die Speiseröhre (29), den Magen (28) oder einen Übergangsbereich zwischen der Speiseröhre (29) und dem Magen (28) legbar ist und das mit dem Hüllteil (20, 20') ebenfalls verbundene oder verbindbare zweite Band (2) distal des ersten Bandes (1) und im Abstand von diesem um den Magen (28) legbar ist und der zwischen dem ersten und dem zweiten Band (1, 2) liegende Bereich des Magens (28) vom Hüllteil (20, 20') umhüllbar ist.

2. Einrichtung nach Anspruch 1, **dadurch gekennzeichnet, dass** das Hüllteil (20, 20') im Wesentlichen über die Länge des ersten und des zweiten Bandes (1, 2) mit dem jeweiligen Band (1, 2) durchgehend oder in Abständen verbunden oder verbindbar ist.

3. Einrichtung nach Anspruch 1 oder 2, **dadurch gekennzeichnet, dass** das erste und das zweite Band (1, 2) und das mit dem ersten und zweiten Band (1, 2) verbundene Hüllteil (20, 20') als herstellerseitig vorgefertigte Einheit ausgebildet sind, vorzugsweise untrennbar miteinander verbunden sind.

4. Einrichtung nach Anspruch 3, **dadurch gekennzeichnet, dass** das Hüllteil (20, 20') an den Bändern (1, 2) angeklebt ist, vorzugsweise in den Bereichen der einander zugewandten Längsränder der Bänder (1, 2).

5. Einrichtung nach einem der Ansprüche 1 bis 3, **dadurch gekennzeichnet, dass** das Hüllteil (20, 20') am ersten oder am zweiten Band (1, 2) oder am ersten und am zweiten Band (1, 2) mittels Verschlusselementen (41, 42) befestigt oder befestigbar ist, vorzugsweise verrastet oder verrastbar ist.

6. Einrichtung nach einem der Ansprüche 1 bis 5, **dadurch gekennzeichnet, dass** die beiden Umfangsenden (21, 22) des Hüllteils (20, 20') im um den Magen (28) gelegten Zustand der Einrichtung einander benachbart sind oder an die beiden Umfangsenden (21, 22) anschließende Abschnitte des Hüllteils (20, 20') einander überlappen und zur Ausbildung einer umfangsgeschlossenen Manschette die beiden benachbarten Umfangsenden (21, 22) oder die an die beiden Umfangsenden (21, 22) anschließenden überlappenden Abschnitte aneinander befestigbar sind.

7. Einrichtung nach Anspruch 6, **dadurch gekennzeichnet, dass** zur gegenseitigen Befestigung der Umfangsenden (21, 22) oder der an die Umfangsenden (21, 22) anschließenden überlappenden Abschnitte des Hüllteils (20, 20') diese miteinander vernähbar sind, vorzugsweise durch Nähösen (23).

8. Einrichtung nach Anspruch 6 oder 7, **dadurch gekennzeichnet, dass** zur gegenseitigen Befestigung der Umfangsenden (21, 22) oder der an die Umfangsenden (21, 22) anschließenden überlappenden Abschnitte des Hüllteils (20, 20') eine Verschlusseinrichtung vorhanden ist, welche vorzugsweise von miteinander verrastbaren Verschlusselementen (37, 38) gebildet wird.

9. Einrichtung nach einem der Ansprüche 1 bis 8, **dadurch gekennzeichnet, dass** das erste Band (1) mindestens eine mit einem Füllmedium befüllbare innere Hohlkammer (15) aufweist, die sich zumindest über einen Großteil der Länge des ersten Bandes (1) erstreckt, wobei durch eine Befüllung der Hohlkammer (15) der innere Durchmesser des vom geschlossenen ersten Band (1) gebildeten Ringes verringerbar ist.

10. Einrichtung nach einem der Ansprüche 1 bis 9, **dadurch gekennzeichnet, dass** das zweite Band (2) mindestens eine mit einem Füllmedium befüllbare innere Hohlkammer (15) aufweist, die sich zumindest über einen Großteil der Längserstreckung des zweiten Bandes (2) erstreckt, wobei durch eine Befüllung der Hohlkammer (15) der innere Durchmesser des vom geschlossenen zweiten Band (2) gebildeten Rings verringerbar ist.

11. Einrichtung nach Anspruch 9 und 10, **dadurch gekennzeichnet, dass** die inneren Hohlkammern (15) des ersten und zweiten Bandes (1, 2) mittels eines gemeinsamen Injektionsports (25) befüllbar sind.

12. Einrichtung nach Anspruch 11, **dadurch gekennzeichnet, dass** die inneren Hohlkammern (15) des ersten und zweiten Bandes (1, 2) frei miteinander kommunizieren.

13. Einrichtung nach Anspruch 11, **dadurch gekennzeichnet, dass** die inneren Hohlkammern (15) des ersten und zweiten Bandes (1, 2) über eine erste und eine zweite Verbindungsleitung (31, 32) miteinander verbunden sind, wobei in der ersten Verbindungsleitung (31) ein bei Übersteigen einer Schließkraft für vom ersten zum zweiten Band (1, 2) fließendes Füllmedium sich öffnendes Rückschlagventil (33) und in der zweiten Verbindungsleitung (32) ein bei Übersteigen einer Schließkraft für vom zweiten zum ersten Band (2, 1) fließendes Füllmedium sich öffnendes Rückschlagventil (35) vorhanden ist.

14. Einrichtung nach einem der Ansprüche 1 bis 13, **dadurch gekennzeichnet, dass** das Hüllteil (20, 20') netzartig ausgebildet ist oder von einer Folie gebildet wird.

15. Einrichtung nach einem der Ansprüche 1 bis 14, **dadurch gekennzeichnet, dass** die Einrichtung weiters ein distal des zweiten Bandes (2) und im Abstand zu diesem ringförmig um den Magen (28) legbares drittes Band (39) mit einer Verschlusseinrichtung (4, 5) zum Schließen des dritten Bandes (39) zu einem Ring aufweist, wobei der zwischen dem zweiten und dritten Band (2, 39) liegende Bereich des Magens (28) vom über das zweite Band (2) bis zum dritten Band (39) sich erstreckenden und mit dem dritten Band (39) verbundenen oder verbindbaren Hüllteil (20, 20') oder von einem weiteren mit dem zweiten und dem dritten Band (2, 39) verbundenen oder verbindbaren Hüllteil (40) umhüllbar ist, wobei vorzugsweise das erste, zweite und dritte Band (1, 2, 39) und das mindestens eine Hüllteil (20, 20', 40) als herstellerseitig vorgefertigte Einheit ausgebildet sind, besonders bevorzugt untrennbar miteinander verbunden sind.

## Claims

1. A device for the treatment of obesity, which comprises a band (1, 2), able to be placed annularly around the stomach (28), with a closing device (4, 5) to close the band (1, 2) to form a ring and comprises an enveloping part (20, 20'), connected or connectable to the band (1, 2), to envelop a stomach region adjoining the applied band (1, 2), **characterised in that** the device comprises at least a first and a second band (1, 2) each with a closing device (4, 5) to close the respective band (1, 2) to form a ring, wherein the first band (1), connected or connectable to the enveloping part (20, 20'), can be placed around the gullet (29), the stomach (28) or a transition region between the gullet (29) and the stomach (28) and the second band (2), likewise connected or connectable to the enveloping part (20, 20'), can be placed around the stomach (28) so as to be distal in relation to the first band (1) and at a distance therefrom and the region, of the stomach (28), lying between the first and the second band (1, 2) can be enveloped by the enveloping part (20, 20').

2. A device according to claim 1, **characterised in that** substantially over the length of the first and the second band (1, 2), the enveloping part (20, 20') is connected or connectable to the respective band (1, 2) in a continuous manner or at intervals.

3. A device according to claim 1 or 2, **characterised in that** the first and the second band (1, 2) and the enveloping part (20, 20') connected to the first and second band (1, 2) are designed as a unit prefabricated by the manufacturer, preferably inseparably connected to one another.

4. A device according to claim 3, **characterised in that** the enveloping part (20, 20') is stuck on the bands (1, 2), preferably in the regions of the facing longitudinal edges of the bands (1, 2).

5. A device according to any one of claims 1 to 3, **characterised in that** the enveloping part (20, 20') is secured or securable to the first or the second band (1, 2) or to the first and the second band (1, 2) by means of closing elements (41, 42), preferably is locked or lockable.

6. A device according to any one of claims 1 to 5, **characterised in that** the two circumferential ends (21, 22) of the enveloping part (20, 20') are adjacent to one another in the state of the device in which it is placed around the stomach (28) or portions, adjoining the two circumferential ends (21, 22), of the enveloping part (20, 20') overlap one another and, in order to form a circumferentially-closed sleeve, the two adjacent circumferential ends (21, 22) or the overlapping portions adjoining the two circumferential ends (21, 22) are securable to one another.

7. A device according to claim 6, **characterised in that** to mutually secure the circumferential ends (21, 22) or the overlapping portions, adjoining the circumferential ends (21, 22), of the enveloping part (20, 20'), these can be sewn to one another, preferably by sewing eyes (23).

8. A device according to claim 6 or 7, **characterised in that** to mutually secure the circumferential ends (21, 22) or the overlapping portions, adjoining the circumferential ends (21, 22), of the enveloping part (20, 20') there is present a closing device, preferably formed by closing elements (37, 38) lockable to one another.

9. A device according to any one of claims 1 to 8, **characterised in that** the first band (1) has at least one inner hollow chamber (15) fillable with a filling medium, which hollow chamber extends over at least a majority of the length of the first band (1), wherein the inner diameter of the ring formed by the closed first band (1) can be decreased by a filling of the hollow chamber (15).

10. A device according to any one of claims 1 to 9, **characterized in that** the second band (2) has at least one inner hollow chamber (15) fillable with a filling medium, which hollow chamber extends over at least a majority of the longitudinal extension of the second band (2), wherein the inner diameter of the ring formed by the closed second band (2) can be decreased by a filling of the hollow chamber (15).

11. A device according to claim 9 and 10, **characterised in that** the inner hollow chambers (15) of the first and second band (1, 2) can be filled by means of a common injection port (25).

12. A device according to claim 11, **characterised in that** the inner hollow chambers (15) of the first and second band (1, 2) communicate freely with one another.

13. A device according to claim 11, **characterized in that** the inner hollow chambers (15) of the first and second band (1, 2) are connected to one another via a first and a second connecting line (31, 32), wherein in the first connecting line (31) there is present a non-return valve (33) opening, when a closing force is exceeded, for filling medium flowing from the first to the second band (1, 2) and in the second connecting line (32) there is present a non-return valve (35) opening, when a closing force is exceeded, for filling medium flowing from the second to the first band (2, 1).

14. A device according to any one of claims 1 to 13, **characterised in that** the enveloping part (20, 20') is net-like or is formed by a film.

15. A device according to any one of claims 1 to 14, **characterised in that** the device furthermore has a third band (39), able to be placed annularly around the stomach (28) so as to be distal in relation to the second band (2) and at a distance therefrom, with a closing device (4, 5) to close the third band (39) to form a ring, wherein the region, of the stomach (28), lying between the second and third band (2, 39) can be enveloped by the enveloping part (20, 20') extending beyond the second band (2) as far as the third band (39) and connected or connectable to the third band (39) or by another enveloping part (40) connected or connectable to the second and the third band (2, 39), wherein preferably the first, second and third band (1, 2, 39) and the at least one enveloping part (20, 20', 40) are in the form of a unit prefabricated by the manufacturer, particularly preferably are inseparably connected to one another.

## Revendications

1. Dispositif de traitement de l'obésité comportant une bague (1, 2) pouvant être positionnée en formant un anneau autour de l'estomac (28) munie d'un dispositif de fermeture (4, 5) permettant de fermer cette bande (1, 2) pour obtenir un anneau, et d'une partie enveloppante (20, 20') reliée ou pouvant être reliée à la bande (1, 2) pour permettre d'envelopper une zone de l'intestin contigüe à la bande (1, 2) positionnée,
**caractérisé en ce que**
le dispositif comporte au moins une première et une seconde bande (1, 2) comportant respectivement un dispositif de fermeture (4, 5) pour permettre de fermer la bande (1, 2) respective pour former un anneau, la première bande (1) reliée ou pouvant être reliée à la partie enveloppante (20, 20') pouvant être positionnée autour de l'oesophage (29), de l'estomac (28) ou d'une zone de transition entre l'oesophage (29) et l'estomac (28), tandis que la seconde bande (2) également reliée ou pouvant également être reliée avec la partie enveloppante (20, 20') peut être positionnée dans une position distale par rapport à la première bande (1) et à distance de celle-ci autour de l'estomac (28), et la zone de l'estomac (28) positionnée entre la première et la seconde bande (1, 2) pouvant être enveloppée par la partie enveloppante (20, 20').

2. Dispositif conforme à la revendication 1,
**caractérisé en ce que**
la partie enveloppante (20, 20') est reliée ou peut être reliée essentiellement sur la longueur de la première et de la seconde bande (1, 2) avec la bande (1, 2) respective de manière continue ou à distance.

3. Dispositif conforme à la revendication 1 ou 2,
**caractérisé en ce que**
la première et la seconde bande (1, 2) et la partie enveloppante (20, 20') reliée à la première et à la seconde bande (1, 2) sont réalisées par le fabricant sous la forme d'une unité préfabriquée, et sont de préférence reliées entre elles de façon inamovible.

4. Dispositif conforme à la revendication 3,
**caractérisé en ce que**
la partie enveloppante (20, 20') est collée aux bandes (1, 2), de préférence dans les zones des bords longitudinaux tournés les uns vers les autres des bandes (1, 2).

5. Dispositif conforme à l'une des revendications 1 à 3,
**caractérisé en ce que**
la partie enveloppante (20, 20') est fixée ou peut être fixée sur la première ou sur la seconde bande (1, 2) ou sur la première et sur la seconde bande (1, 2) au moyen d'éléments de fermeture (41, 42), et est de préférence encliquetée ou est de préférence encliquetable.

6. Dispositif conforme à l'une des revendications 1 à 5,
**caractérisé en ce que**
lorsque le dispositif est positionné autour de l'estomac (28) les deux extrémités périphériques (21, 22) de la partie enveloppante (20, 20') sont contigües ou se chevauchent sur les zones de la partie enveloppante (20, 20') voisines des deux extrémités périphériques (21, 22), et, pour permettre de former une manchette fermée sur sa périphérie, les deux extrémités périphériques (21, 22) voisines ou les tronçons se chevauchant voisins des deux extrémités périphériques (21, 22) peuvent être fixés les uns aux autres.

7. Dispositif conforme à la revendication 6,
**caractérisé en ce que**
pour permettre la fixation réciproque des extrémités périphériques (21, 22) ou des tronçons de la partie enveloppante (20, 20') se chevauchant voisins des extrémités périphériques (21, 22), ces éléments peuvent être cousus, de préférence au moyen d'oeillets de couture (23).

8. Dispositif conforme à la revendication 6 ou 7,
**caractérisé en ce que**
pour permettre la fixation réciproque des extrémités périphériques (21, 22) ou des tronçons se chevauchant voisins des extrémités périphériques (21, 22) de la partie enveloppante (20, 20') il est prévu un dispositif de fermeture qui est de préférence formé par des éléments de fermeture encliquetables (37, 38).

9. Dispositif conforme à l'une des revendications 1 à 8,
**caractérisé en ce que**
la première bande (1) comporte au moins une chambre creuse interne (15) pouvant être remplie d'un fluide de remplissage qui s'étend sur au moins une grande partie de la longueur de la première bande (1), le remplissage de cette chambre creuse (15) permettant de diminuer le diamètre interne de l'anneau formé par la première bande (1) fermée.

10. Dispositif conforme à l'une des revendications 1 à 9,
**caractérisé en ce que**
la seconde bande (2) comporte au moins une chambre creuse interne (15) pouvant être remplie d'un fluide de remplissage qui s'étend sur au moins une grande partie de l'étendue longitudinale de la seconde bande (2), le remplissage de cette chambre creuse (15) permettant de diminuer le diamètre interne de l'anneau formé par la seconde bande (2) fermée.

11. Dispositif conforme à la revendication 9 et 10,
**caractérisé en ce que**
les chambres creuses internes (15) de la première et de la seconde bande (1, 2) peuvent être remplies au moyen d'un orifice d'injection commun (25).

12. Dispositif conforme à la revendication 11,
**caractérisé en ce que**
les chambres creuses internes (15) de la première et de la seconde bande (1, 2) communiquent librement entre elles.

13. Dispositif conforme à la revendication 11,
**caractérisé en ce que**
les chambres creuses internes (15) de la première et de la seconde bande (1, 2) sont reliées entre elles par l'intermédiaire d'une première et d'une seconde conduite de liaison (31, 32), la première conduite de liaison (31) étant équipée d'une soupape anti-retour (33) s'ouvrant lorsque le fluide de remplissage s'écoulant de la première à la seconde bande (1, 2), dépasse d'une force de fermeture et la seconde conduite de liaison (32) étant équipée d'une soupape anti-retour (35) s'ouvrant lorsque le fluide de remplissage s'écoulant de la seconde à la première bande (1, 2) dépasse d'une force de fermeture.

14. Dispositif conforme à l'une des revendications 1 à 13,
**caractérisé en ce que**
la partie enveloppante (20, 20') est réalisée en forme de réseau ou est formée par une feuille.

15. Dispositif conforme à l'une des revendications 1 à 14,
**caractérisé en ce qu'**
il est en outre équipé d'une troisième bande (39) pouvant être positionnée autour de l'estomac (28) en formant un anneau, dans une position distale par rapport à la seconde bande (2) et à distance de celle-ci, munie d'un dispositif de fermeture (4, 5) pour permettre de fermer la troisième bande (39) de manière à obtenir un anneau, la zone de l'estomac (28) située entre la seconde et la troisième bande (2, 39) pouvant être entourée par la partie enveloppante (20, 20') s'étendant de la seconde bande (2) jusqu'à la troisième bande (39 et reliée ou pouvant être reliée à la troisième bande (39), ou par une autre partie enveloppante (40) reliée ou pouvant être reliée avec la seconde et la troisième bande (2, 39), la première, la seconde et la troisième bande (1, 2, 39) et la partie enveloppante (20, 20', 40) étant de préférence réalisées sous la forme d'une unité préfabriquée au niveau du fabricant, et de façon particulièrement préférentielle reliées entre elles de façon inamovible.
